(19) Europäisches Patentamt
European Patent Office
Office européen des brevets

(11) Publication number: **0 281 000 B1**

(12) **EUROPEAN PATENT SPECIFICATION**

(45) Date of publication of patent specification: **16.10.91** (51) Int. Cl.5: **G01N 33/70**, G01N 33/52

(21) Application number: **88102645.4**

(22) Date of filing: **23.02.88**

(54) **Multi-layer analytical element for creatinine analysis.**

(30) Priority: **27.02.87 JP 42820/87**

(43) Date of publication of application:
**07.09.88 Bulletin 88/36**

(45) Publication of the grant of the patent:
**16.10.91 Bulletin 91/42**

(84) Designated Contracting States:
**BE CH DE FR GB IT LI NL SE**

(56) References cited:
**EP-A- 0 137 521**
**DE-A- 3 240 463**

**CHEMICAL ABSTRACTS, vol. 92, no. 21, 26 May 1980, Columbus, OH (US); p. 295, no. 177055x**

**ANALYTICAL CHEMISTRY, vol. 55, no. 4, April 1983, American Chemical Society (US); B.WALTER, p. 498a-514a**

**CHEMICAL ABSTRACTS, vol. 99, 1983, Columbus, OH (US); p. 243-244, no. 49982e**

**CHEMICAL ABSTRACTS, vol. 98, 1983, Columbus, OH (US); M.W.SUNDBERG et al., p. 284, no. 212342m**

**CHEMICAL ABSTRACTS, vol. 88, 1978, Columbus, OH (US); T.R.FISHER et al., p. 271, no. 18533c**

**CLINICAL CHEMISTRY, vol. 26, no. 8, July 1980, Winston-Salem, NC (US); S.NARAYANAN et al., p. 1119-1126**

(73) Proprietor: **KONICA CORPORATION**
**26-2, Nishishinjuku 1-chome, Shinjuku-ku**
**Tokyo 160(JP)**

(72) Inventor: **Koyama, Mikio, c/o Konica Corporation**
**1, Sakuramachi**
**Hino-shi, Tokyo(JP)**
Inventor: **Akashi, Tomoji, c/o Konica Corporation**
**1, Sakuramachi**
**Hino-shi, Tokyo(JP)**

(74) Representative: **Henkel, Feiler, Hänzel & Partner**
**Möhlstrasse 37**
**W-8000 München 80(DE)**

**EP 0 281 000 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

BACKGROUND OF THE INVENTION

This invention relates to a multi-layer analytical element for analysis of creatinine in a liquid sample.

Measurement of creatinine is increasing its importance as a part of kidney function diagnosis in recent years. For measurement of creatinine, chemical methods such as the Jaffe method, etc. have been frequently used, but these methods are complicated requiring use of a highly alkaline solution and further protein removal operation, and have also the drawback that creatine existing in the body fluid is measured as a positive error.

On the other hand, the measuring method by use of an enzyme with higher reaction specificity, although it is more or less simplified in operation, cannot but be influenced by various endogenous substances depending on the enzyme used. Particularly, it has been known to have a large error along with the complicatedness in removal operation of endogenous substances during measurement of a sample with extremely small existing concentration such as blood.

In recent years, even in the field of dry analysis which is becoming popular, this problem is not changed at all. For example, it is clear in the case of performing measurement by use of the methods as disclosed in Japanese Unexamined Patent Publications Nos. 3488/1977 and 72692/1981. According to the descriptions in the above specifications, a first reagent layer, a semipermeable membrane layer and a second reagent layer are constituted on a support. In the mode conventionally used, a porous spreading layer is further laminated. In the first layer, a pH indicator is contained, creatinine diminase is contained in the second layer, and the liquid sample supplied from the porous spreading layer is decomposed according the following reaction scheme.

$$\text{Creatinine} \xrightarrow{\text{Creatinine diminase}} \text{N-methylhydantoin} + NH_3$$

Of ammonia and N-methylhydantoin formed in this reaction, only ammonia is permitted to permeate through the semipermeable membrane layer into the first reagent layer, which ammonia reacts with the pH indicator to exhibit a detectable response.

This may be apparently a simple method, but since ammonia in the body fluid is also similarly measured, measurement of creatinine is accompanied necessarily with mesurement of ammonia, thus having the drawback that genuine value of creatinine must be determined from the difference in those results.

The same is the case in the reaction of using creatininase, creatinase, sarcosine oxidase for measuring hydrogen peroxide, and it is necessary to remove endogenous creatine by some methods.

According to Chemical Abstracts, vol. 92, no.177055x (1980) creatinine and creatine are quantitatively detected by treating

a) creatinine with creatinine amidohydrolase, creatine amidinohydrolase, and sarcosine dehydrogenase in the presence of a catalyst and

b) creatine with creatine amidinohydrolase and sarcosine dehydrogenase in the presence of a catalyst to form HCHO.

The HCHO formed is detected spectrometrically to measure creatinine and creatine.

SUMMARY OF THE INVENTION

An object of the present invention is to provide a multi-layer analytical element for analysis of creatinine free from these drawbacks in the prior art.

To outline the present invention, the present invention is an invention concerning a multi-layer analytical element for analysis of creatinine, having a reagent layer on a support, a porous spreading layer thereabove, and containing at least creatininase, creatinase, sarcosine oxidase and a composition for detecting hydrogen peroxide, wherein said creatinase is contained in the layer nearer to the spreading layer than the creatininase-containing layer and/or is contained in the spreading layer, and wherein said element contains a buffering agent in an amount sufficient to regulate the pH within the element so as to make the

activity of said creatinase higher than that of said creatininase.

The present invention also concerns a method for analysing creatinine in a liquid sample which comprises employing a multi-layer analytical element described above.

## DESCRIPTION OF PREFERRED EMBODIMENTS OF THE INVENTION

The reactions to be used in the present invention follow the reaction schemes shown below.

$$\text{Creatinine} \xrightarrow{\text{Creatininase}} \text{Creatine}$$

$$\text{Creatine} \xrightarrow{\text{Creatinase}} \text{Sarcosine} + \text{urea}$$

$$\text{Sarcosine} + O_2 + H_2O \xrightarrow{\text{Sarcosine oxidase}} H_2O_2 + \text{Glycine} + \text{Formaldehyde}$$

As described above, it is necessary to separate the creatine formed from creatinine and from the creatine existing in a liquid sample, for example, blood.

For this purpose, in the present invention, said creatinase is contained in the layer nearer to said spreading layer than the creatininase-containing layer and/or is contained in the spreading layer. Namely, the enzyme with which the liquid sample supplied from the porous spreading layer is first brought into contact is creatinase, which is intended to be reacted with endogenous creatine at the initial stage of the reaction.

In the analytical element of the present invention, a buffering agent is contained in the element preferably in an amount sufficient to regulate the pH within the element to 6.5 to 8.0.

Namely, the reason why the pH within the element is preferably regulated to 6.5 to 8.0 is that the activity of creatinase is enhanced as compared with that of creatininase, thereby effecting removal of endogenous creatine.

Further, in some embodiments of the present invention, the creatinase is contained in a larger amount than the creatininase.

More specifically, for permitting the reactions of creatininase and creatinase to proceed without delay, it is preferred that the contents of creatininase and creatinase should be such as to obtain an activity for creatinase which is 1.2 to 5.0-fold that of creatininase, which latter is chosen as 1 in terms of International Units.

This is because the enzymatic reaction catalyzed by creatininase is an equilibrium reaction, in which the reaction proceeds in the reverse direction unless creatinine in the liquid sample is converted with creatininase to creatine, which creatine is decomposed rapidly with creatinase to sarcosine and urea, whereby the reaction as a whole can proceed with difficultly, consequently exhibiting only poor response.

Also, as another advantage, since creatinase is contained in 1.2 to 5.0-fold International Unit amount based on creatininase, removal of endogenous creatine can be accomplished in further shorter time.

The analytical element of the present invention is provided preferably in the form laminated on a transparent water impermeable support. Such support of the present invention should be preferably transparent and water impermeable, as exemplified by polyethyleneterephthalate, polycarbonate, cellulose acetate, polystyrene, pclyethylene, etc. The film thickness of the support used can be selected suitably as desired from about 50 $\mu$m to 500 $\mu$m.

The surface on which the support according to the present invention is laminated should be preferably applied with the treatment for improvement of adhesiveness with the layer, if desired. For example, there may be employed physical treatment such as flame treatment and corona discharging treatment, coating of a subbing layer for improvement of adhesiveness, and further combinations of these, etc.

In the present invention, creatinase is contained in the spreading layer/and or a layer nearer to the spreading layer than creatininase-containing layer, as described above.

More specifically, in the case of an analytical element of the present invention having a first reagent layer, a second reagent layer and a porous spreading layer laminated from the support side, the first embodiment comprises a support, a first reagent layer containing creatininase, a second reagent layer containing creatinase and a porous spreading layer.

The second embodiment comprises a first reagent layer containing creatininase on a support, a second reagent layer containing creatinase and a porous spreading layer containing creatinase.

Another embodiment comprises a reagent layer containing creatininase on a support and a porous spreading layer containing creatinase.

As other reagents to be contained in the reagent layer, there are sarcosine oxidase and a composition for detection of hydrogen peroxide. The composition for detection of hydrogen peroxide comprises a substance having peroxidase action and chromogen, for example, a hydrogen donor and a coupler. As the substance having peroxidase action, peroxidase, hemoglobin, hematin, etc. may be included, preferably peroxidase.

As other enzymes contained, there are sarcosine oxidase and peroxidase, and these may be in any of the above layers, and also still another layer may be provided and they can be contained therein. Preferably, they should be contained together with creatininase.

For creatininase, creatinase, sarcosineoxidase, peroxidase to be used in the present invention, broad contents can be selected depending on the purpose.

Creatininase can be selected within the range from 3,000 International Unit (hereinafter abbreviated as IU)/$m^2$ or more, preferably 5,000 IU/$m^2$ to 500,000 IU/$m^2$, creatinase from 6,000 IU/$m^2$ or more, preferably from 6,000 IU/$m^2$ to 2,500,000/$m^2$, sarcosine oxidase from 5,000 IU/$m^2$ to 50,000 IU/$m^2$, and peroxidase from 5,000 IU/$m^2$ to 100,000 IU/$m^2$.

However, in the case of the rate method by observation of the concentration per unit time, creatininase should be preferably from 5,000 IU/$m^2$ to 100,000 IU/$m^2$, and creatinase from 6,000 IU/$m^2$ to 500,000 IU/$m^2$.

On the other hand, in the case of the end point method, creatinase should be preferably from 120,000 IU/$m^2$ to 2,500,000 IU/$m^2$, and creatininase from 100,000 IU/$m^2$ to 500,000 IU/$m^2$.

As the binder in the reagent layer, hydrohilic colloidal substances are preferred. Examples of hydrophilic colloidal substance may include gelatin, gelatin derivatives (e.g. phthalated gelatin, etc.), agarose, alginic acid, water-soluble cellulose derivatives (e.g. hydroxyethyl cellulose, carboxymethyl cellulose sodium salt, methyl cellulose, ethyl cellulose, etc.), polyacrylamides (e.g. polyacrylamide, polymethacrylamide, and copolymers thereof), polyvinyl pyrrolidone and copolymers thereof, polyvinyl alcohol, etc.

The chromogen can be contained in any of the layers in the analytical element, but should preferably be contained in the same layer in which peroxidase is contained. As the chromogen to be used in the present invention, any compound capable of exhibiting detectable response with peroxidase can be used. Detectable response as herein mentioned may include color forming reaction, color extinguishing reaction, fluorescence emission reaction, light emission reaction, etc. Preferably, color forming reaction may be employed.

For example, there may be included a combination of 4-aminoantipyrine derivative known as Trinder reagent with 1-naphthol derivative, combinations of the compounds as disclosed in Japanese Unexamined Patent Publications Nos. 94653/1982, 94654/1982, 94655/1982 and 94656/1982, dye forming precursors as disclosed in Japanese Unexamined Patent Publication No. 26188/1978.

Further, 4-alkoxy-1-naphthols, ABTS [2,2'-azino-bis(3-ethylbenzothiazoline-6-sulfonic acid], TMB (tetramethylbenzidine), etc. can be also used.

The above coupler and hydrogen donor can be contained easily in the reagent layer in the form of the so called direct dispersion by dissolving directly in water or dissolving in an organic solvent, alternatively according to the method such as the oil-protect dispersion, etc.. In case where a coupler is contained in the reagent layer in the oil-protected form, storage stability of the analytical element can be improved.

In the reagent layer, a bufferng agent for buffering at preferably pH 6.5 to 8.0 is contained. The buffering agent may be one capable of buffering the above range, including various ones such as phosphate buffering agent ($KH_2PO_4$-$K_2HPO_4$) and those known as Good buffering agents [e.g. TES{N-tris-(hydroxymethyl)methyl-2-aminoethanesulfonic acid}-KOH, TAPS{N-tris(hydroxymethyl)methyl-3-aminopropanesulfonic acid}-KOH]

For the porous spreading layer according to the present invention, various known ones may be available. For example, there may be included nonfibrous porous medium layer disclosed in Japanese Patent Publication No. 21677/1978, the so called brush polymer layer, the particulate transporting member comprising particles and adhesive fine particles disclosed in Japanese Unexamined Patent Publication No. 90859/1980, the fabric subjected to hydrophilic treatment disclosed in Japanese Unexamined Patent Publication No. 164356/1980, the fibrous spreading layer comprising loose fibers and a reactive polymer

disclosed in Japanese Unexamined Patent Publication No. 197466/1982, the bound product of particles having particles bound directly or through a low molecular weight compound disclosed in Japanese Unexamined Patent Publications Nos. 101760/1982 and 101761/1982.

Particularly, the spreading layer disclosed in Japanese Unexamined Patent Publication No. 197466/1982 is useful as one which can be prepared easily and can give sufficient precision.

The porous spreading layer according to the present invention may have a film thickness of from about 50 µm to about 1 mm, preferably from about 100 µm to about 500 µm.

Further, of porous spreading layers, loose fibers (referred to as fibers for filter paper raw material) to be used in the fibrous spreading layer disclosed in the above Japanese Unexamined Patent Publication No. 197466/1982 can have wide range of sizes from 40 mesh to 500 mesh according to the JIS sieve.

According to one embodiment of the present invention, creatinase can be contained in the porous spreading layer. Assembling an enzyme stably in the spreading layer can be accomplished by lyophilizing the above enzyme together with a protein which does not inhibit said enzyme according to the disclosure in Japanese Unexamined Patent Publication No. 177997/1986 and containing the lyophilized powder as fine powder.

Further, when combinations as disclosed in Japanese Unexamined Patent Publications Nos. 94653/1982, 94654/1982, 94655/1982 and 94656/1982 are used as the chromogen, it is useful to incorporate a hydrogen donor.

The multi-layer analytical element of the present invention comprises a reagent layer and a porous spreading layer as the essential constituent layers, but other functional layer can be also assembled, if necessary.

Examples of other functional layer may include light reflection layer, filtration layer, barrier layer, migration impeding layer, registration layer, obstructive material elmininating layer, etc. or structural layer (e.g. adhesive layer, etc.).

In the respective layers of the multi-layer analytical element of the present invention, various additives can be added. For example, surfactants (anionic, cationic, amphoteric, nonionic surfactants), film hardening agents, preservatives (e.g. ethylenediaminetetraacetic acid, etc.) can be contained. The surfactant may be employed not only for the purpose of spreading effect in the coating of the respective steps, but also for the effect of an aid for dispersion, etc. On the other hand, the film hardening agent can crosslink the hydrophilic binder in the reagent layer, thereby preventing migration of the reagents, etc. into the spreading layer in the incubation process after application of a liquid sample onto the analytical element. Further, as the preservative, for example, ethylenediaminetetraacetic acid or its salt which can trap the substance interfering with enzymatic activity may be effectively used.

In the multi-layer analytical element of the present invention, a biological liquid sample, namely blood, plasma, serum, urea, cerebospinal fluid, etc. may be used. The amount used may be from 5 µl to 20 µl, preferably from 7 µl to 15 µl. Generally, 10 µl is preferred.

The measuring wavelength may be determined depending on the dye generated from the chromogen, but selected in the visible range, namely within the range from 400 nm to 700 nm. Preferably, it should be selected from the wavelength of 450 nm or longer. As the preferable measuring method, there may be employed the so called rate method, in which from the point on completion of the reaction of endogenous creatine, multiple measurements are conducted for a certain time to determine the concentration change amounts per unit time therebetween, and the so called two point measuring method in which measurement is conducted similarly on completion of the reaction of endogenous creatine, and further the measurement is conducted again after a certain time to determine the concentration between the two points.

The present invention is described in more detail by referring to Examples, by which the present invention is not limited at all.

In the respective Examples, creatinase and creatininase employed were derived from Flavobacterium sp., sarcosine oxidase from Corynebacterium sp. and peroxidase from horseradish.

Example - 1

On a transparent polyethyleneterephthalate film subjected to subbing with a thickness of about 180 µm were laminated layers having the following compositions successively in this order to prepare analytical element (1) of the present invention..

(First reagent layer)

Deionized gelatin 10 g/$m^2$

Peroxidase 7000 IU/$m^2$
Sarcosine oxidase 15000 IU/$m^2$
Creatininase 100000 IU/$m^2$
Dibutyl phthalate 6.7 g/$m^2$
1-(2,4,6-trichlorophenyl)-3-(2-chloro-octadecylsuccinimidoanilino)-5-pyrazolone 6.7 g/$m^2$
1.8M potassium phosphate buffer (pH 7.5) 38.0 g/$m^2$
Alkanol XC (commercially available from Du Pont Co.) 0.15 g/$m^2$
Bis(vinylsulfonylmethyl) ether 0.36 g/$m^2$

(Second reagent layer):

Deionized gelatin 5 g/$m^2$
Creatinase 125000 IU/$m^2$
Ascorbate oxidase 10000 IU/$m^2$
1.8M potassium phosphate buffer (pH 7.5) 19.0 g/$m^2$
Alkanol XC 0.075 g/$m^2$
Bis(vinylsulfonylmethyl)ether 0.18 g/$m^2$
Disodium ethylenediaminetetraacetate 0.56 g/$m^2$

(Adhesive layer):

Vinyl pyrrolidone/vinyl acetate copolymer (polymerization ratio 2/8) 1.25 g/$m^2$

(Porous spreading layer):

Fiber for filter paper raw material (40 mesh or higher) 105 g/$m^2$
Triton X-100 (Rohm & Haas Co.) 10.2 g/$m^2$
N,N-diethyl-3-methanesulfonamidoethyl-4-aminoanilin-p-toluenesulfonic acid 0.15 g/$m^2$
Styrene/glycidyl methacrylate copolymer (copolymerization ratio 9/1) 25.56 g/$m^2$.

Similarly, on the same transparent polyethyleneterephthalate support subjected to subbing with a thickness of 180 μm was laminated a reagent layer having the following composition to prepare comparative analytical element (1).

(Reagent layer)

Gelatin 10 g/$m^2$
Peroxidase 700 IU/$m^2$
Sarcosine oxidase 15000 IU/$m^2$
Creatinase 125000 IU/$m^2$
Creatininase 100000 IU/$m^2$
1-(2,4,6-trichlorophenyl)-3-(2-chloro-octadecylsuccinimidoanilino)-5-pyrazolone 6.7 g/$m^2$
Dibutyl phthalate 6.7 g/$m^2$
1.8M potassium phosphate buffer (pH 7.5) 38.0 g/$m^2$
Ascorbate oxidase 10000 IU/$m^2$
Disodium ethylenediaminetetraacetate 0.56 g/$m^2$
Alkanol XC 0.15 g/$m^2$
Bis(vinylsulfonylmethyl) ether 0.36 g/$m^2$

Further, the adhesive layer and the porous spreading layer shown in the above analytical element (1) of the present invention were laminated with the same compositions to prepare a comparative analytical element (1). Standard solutions containing creatinine and creatine dissolved by mixing at various concentrations were prepared, and these were subjected to measurement of creatinine by the enzymatic method [CRE(E) KAINOS., commercially available from KAINOS K.K.] and creatine by the picric acid salt method to determine the concentrations of both, followed by spotting of 10 μl of the test sample onto the analytical element (1) of the present invention and the comparative analytical element (1) to carry out incubation at 37 °C, and the reflective densities 3 minutes and 7 minutes after spotting were measured by use of a filter of 546 nm, and the reflective density difference ($\Delta D_R$) between 7 minutes and 3 minutes and the reflective

density after 7 minutes were calculated. The results are shown in Table 1.

Table 1

| Test Sample No. | | 1 | 2 | 3 | 4 | 5 | 6 | 7 | 8 | 9 | 10 |
|---|---|---|---|---|---|---|---|---|---|---|---|
| Creatinine (mg/dl) | | 1.0 | 1.0 | 3.0 | 3.0 | 5.0 | 5.0 | 10.0 | 10.0 | 15.0 | 15.0 |
| Creatine (mg/dl) | | 0.0 | 0.5 | 0.0 | 1.0 | 0.0 | 3.0 | 0.0 | 5.0 | 0.0 | 7.0 |
| Analytical element (1) | $\Delta D_R$ | 0.068 | 0.068 | 0.163 | 0.164 | 0.242 | 0.243 | 0.402 | 0.405 | 0.512 | 0.516 |
| | $D_R$ | 0.426 | 0.488 | 0.589 | 0.638 | 0.728 | 0.876 | 1.008 | 1.181 | 1.202 | 1.353 |
| Comparative element (1) | $\Delta D_R$ | 0.083 | 0.108 | 0.188 | 0.208 | 0.256 | 0.337 | 0.412 | 0.543 | 0.548 | 0.632 |
| | $D_R$ | 0.455 | 0.506 | 0.651 | 0.710 | 0.784 | 0.936 | 1.062 | 1.260 | 1.290 | 1.459 |

According to Table 1, the analytical element (1) of the present invention is not dependent on the creatine amount by measurement of $D_R$ of 7 min. - 3 min. and has good relationship with the concentration of creatinine. In contrast, in the comparative analytical element (1), it can be clearly seen that positive error

has occured depending on the creatine amount even if $\Delta D_R$ may be taken.

Example - 2

Of the composition of the analytical element of the present invention used in Example - 1, by varying pH of the buffering agent as 6.0, 7.0, 7.8 and 8.5, analytical elements (2) to (5) of the present invention were prepared, and by use of the same creatinine standard solutions as in Example - 1, the difference in $D_R$ - ($\Delta D_R$) between 3 minutes and 7 minutes after spotting were determined. The results are shown in Table 2.

Table 2

| Analytical element No. | pH | Test sample No. 1 | 2 | 3 | 4 | 5 | 6 | 7 | 8 | 9 | 10 |
|---|---|---|---|---|---|---|---|---|---|---|---|
| (2) | 7.0 | 0.058 | 0.059 | 0.147 | 0.148 | 0.229 | 0.233 | 0.391 | 0.395 | 0.506 | 0.508 |
| (3) | 7.8 | 0.069 | 0.070 | 0.168 | 0.167 | 0.253 | 0.255 | 0.418 | 0.420 | 0.514 | 0.518 |
| (4) | 6.0 | 0.011 | 0.015 | 0.032 | 0.035 | 0.052 | 0.054 | 0.103 | 0.108 | 0.136 | 0.141 |
| (5) | 8.5 | 0.073 | 0.089 | 0.173 | 0.202 | 0.284 | 0.332 | 0.477 | 0.586 | 0.611 | 0.697 |

As is apparent from Table 2, analytical elements (2) and (3) in which the pH of the buffering agent was regulated to 7.0 or 7.8, respectively, exhibit particularly excellent effect in removal of an influence of the endogenous creatine.

Example - 3

On a transparent polyethyleneterephthalate support subjected to subbing with a thickness of 180 µm were laminated successively the layers having the following compositions to prepare analytical elements (6) to (9) of the present invention.

(Reagent layer)

Deionized gelatin 10 g/$m^2$
Peroxidase 12000 IU/$m^2$
Creatininase shown in Table 3
Sarcosine oxidase 15000 IU/$m^2$
2-Hydroxy-3,5-dichlorobenzenesulfonic acid 1.2 g/$m^2$
4-Aminoantipyrine hydrochloride 0.8 g/$m^2$
1.8M potassium phosphate buffering agent (pH 7.5) 38.0 g/$m^2$
Sodium ethylenediaminetetraacetate 0.56 g/$m^2$
Bis(vinylsulfonylmethyl)ether 0.36 g/$m^2$

(Adhesive layer)

Vinyl pyrrolidone/vinyl acetate copolymer (copolymerization ratio 2/8) 1.25 g/$m^2$

(Spreading layer)

Fiber for filter paper raw material (40 mesh or more) 105 g/$m^2$
Triton X-100 10.2 g/$m^2$
Creatinase shown in Table 3
Bovine serum albumin (dissoolved together with creatininase and contained as lyophilized powder) 3.8 g/$m^2$
Styrene/glycidyl methacrylate copolymer (copolymerization ratio 9/1) 25.56 g/$m^2$

Table 3

| | Activity amount contained (10,000 IU/$m^2$) | | | |
|---|---|---|---|---|
| Analytical element No. | (6) | (7) | (8) | (9) |
| Creatininase | 10 | 1 | 1 | 1 |
| Creatinase | 15 | 3 | 4.5 | 0.8 |

After the above analytical elements (6) - (9) of the present invention were spotted with 10 µl of the test samples 1 - 8 used in Example - 1, incubation was conducted at 37 °C, and the reflective densities were

measured 3 and 7 minutes fter spotting similarly as in Example 1 by use of a filter of 546 nm. The results are shown in Table 4.

Table 4

| | Test sample No. | | | | | | | |
|---|---|---|---|---|---|---|---|---|
| | 1 | 2 | 3 | 4 | 5 | 6 | 7 | 8 |
| Analytical element (6) | 0.081 | 0.080 | 0.207 | 0.208 | 0.333 | 0.335 | 0.537 | 0.539 |
| Analytical element (7) | 0.090 | 0.090 | 0.246 | 0.247 | 0.368 | 0.372 | 0.5643 | 0.568 |
| Analytical element (8) | 0.093 | 0.093 | 0.288 | 0.289 | 0.429 | 0.433 | 0.611 | 0.615 |
| Analytical element (9) | 0.004 | 0.007 | 0.018 | 0.022 | 0.092 | 0.099 | 0.143 | 0.144 |

As is apparent from Table 4, the analytical elements (6) to (8) in which ratio of the creatinase to the creatininase is 1.2 or more exhibit particularly excellent effect in removal of an influence of the endogenous creatine.

As described above, by use of the analytical element of the preent invention, there can be exhibited a remarkable effect that creatinine can be quantitated by one operation with good precision while excluding the influence by endogenous creatine.

## Claims

1. A multi-layer analytical element for analysis of creatinine in a liquid sample having a reagent layer on a support, a porous spreading layer thereabove, and containing at least creatininase, creatinase, sarcosine oxidase and a composition for detecting hydrogen peroxide, wherein said creatinase is contained in the layer nearer to the spreading layer than the creatininase-containing layer and/or is contained in the spreading layer, and wherein said element contains a buffering agent in an amount sufficient to regulate the pH within the element so as to make the activity of said creatinase higher than that of said creatininase.

2. The analytical element according to claim 1, wherein said creatinase is contained in the spreading layer.

3. The analytical element according to Claim 1 or 2, wherein said composition for detecting hydrogen peroxide comprises a substance having the peroxidase action, a hydrogen donor and a coupler.

4. The analytical element according to Claim 3, wherein said coupler is dispersed in the reagent layer in the oil-protected form.

5. The analytical element according to Claim 1 or 2, wherein said creatinase is contained in an amount such that the activity thereof is higher than the activity of said creatininase.

6. The analytical element according to Claim 5, wherein said composition for detecting hydrogen peroxide comprises a substance having the peroxidase action, a hydrogen donor and a coupler.

7. The analytical element according to one or more of Claims 1 to 6, wherein said creatinase is contained in an amount such that the activity thereof is 1.2-fold or more of the activity of said creatininase.

8. The analytical element according to one or more of Claims 1 to 6, wherein said creatinase is contained in an amount of 6,000 $IU/m^2$ or more and said creatininase is contained in an amount of 3,000 $IU/m^2$ or more.

9. The analytical element according to Claim 8, wherein said creatinase is contained in an amount of from 6,000 $IU/m^2$ to 2,500,000 $IU/m^2$ and said creatininase is contained in an amount of from 5,000 $IU/m^2$ to 500,000 $IU/m^2$.

10. A method for analysing creatinine in a liquid sample which comprises employing a multi-layer analytical element according to claims 1 and 2.

## Revendications

1. Un élément d'analyse à plusieurs couches pour analyse de créatinine dans un échantillon liquide comportant une couche de réactif sur un support, une couche poreuse d' étalement sur celle-ci, et contenant au moins de la créatininase, de la créatinase, de l'oxydase de sarcosine et une composition pour détecter le péroxyde d'hydrogène, dans lequel ladite créatinase est contenue dans la couche plus proche de la couche d'étalement que la couche contenant la créatininase et/ou est contenue dans la couche d'étalement, et dans lequel ledit élément contient un agent tampon suivant une quantité suffisante pour réguler le pH dans l'élément de manière à rendre l'activité de ladite créatinase supérieure à celle de ladite créatininase.

2. L'élément d'analyse selon la revendication 1, dans lequel ladite créatinase est contenue dans la couche d'étalement.

3. L'élément d'analyse selon la revendication 1 ou 2, dans lequel ladite composition pour détecter le

péroxyde d'hydrogène comporte une substance présentant l'action de la péroxydase, un donneur d'hydrogène et un coupleur.

4. L'élément d'analyse selon la revendication 3, dans lequel ledit coupleur est dispersé dans la couche de réactif sous la forme d'une protection par de l'huile.

5. L'élément d'analyse selon la revendication 1 ou 2, dans lequel ladite créatinase est contenue suivant une quantité telle que son activité est supérieure à l'activité de ladite créatininase.

6. L'élément d'analyse selon la revendication 5, dans lequel ladite composition pour détecter le péroxyde d'hydrogène comporte une substance présentant l'action de la péroxydase, un donneur d'hydrogène et un coupleur.

7. L'élément d'analyse selon l'une au moins des revendications 1 à 6, dans lequel ladite créatinase est contenue suivant une quantité telle que son activité est d'au moins 1,2 fois l'activité de ladite créatininase.

8. L'élément d'analyse selon l'une au moins des revendications 1 à 6, dans lequel ladite créatinase est contenue suivant une quantité de 6.000 $UI/m^2$ ou plus et ladite créatininase est contenue suivant une quantité de 3.000 $UI/m^2$ ou plus.

9. L'élément d'analyse selon la revendication 8, dans lequel ladite créatinase est contenue suivant une quantité de 6.000 $UI/m^2$ à 2.500.000 $UI/m^2$, et ladite créatininase est contenue suivant une quantité de 5.000 $UI/m^2$ à 500.000 $UI/m^2$.

10. Un procédé pour analyse de créatinine dans un échantillon liquide, qui comporte l'utilisation d'un élément d'analyse à plusieurs couches selon les revendications 1 et 2.

**Patentansprüche**

1. Mehrlagiges analytisches Element zur Analyse von Creatinin in einer flüssigen Probe mit einer Reagensschicht auf einem Schichtträger, einer darauf befindlichen porösen Verlaufschicht und mit zumindest Creatininase, Creatinase, Sarcosinoxidase und einem Mittel zum Nachweis von Wasserstoffperoxid, wobei die Creatinase in der näher an der Verlaufschicht als die creatininasehaltige Schicht befindlichen Schicht und/oder in der Verlaufschicht enthalten ist und wobei das Element ein Puffermittel in einer Menge, die ausreicht, den pH-Wert innerhalb des Elements derart einzustellen, daß die Aktivität der Creatinase höher wird als diejenige der Creatininase, enthält.

2. Analytisches Element nach Anspruch 1, dadurch gekennzeichnet, daß die Creatinase in der Verlaufschicht enthalten ist.

3. Analytisches Element nach Anspruch 1 oder 2, dadurch gekennzeichnet, daß das Mittel zum Nachweis von Wasserstoffperoxid eine Substanz mit Peroxidasewirkung, einen Wasserstoffdonator und einen Kuppler umfaßt.

4. Analytisches Element nach Anspruch 3, dadurch gekennzeichnet, daß der Kuppler in der Reagensschicht in ölgeschützter Form enthalten ist.

5. Analytisches Element nach Anspruch 1 oder 2, dadurch gekennzeichnet, daß die Creatinase in einer solchen Menge vorhanden ist, daß ihre Aktivität höher ist als diejenige der Creatininase.

6. Analytisches Element nach Anspruch 5, dadurch gekennzeichnet, daß das Mittel zum Nachweis von Wasserstoffperoxid eine Substanz mit Peroxidasewirkung, einen Wasserstoffdonator und einen Kuppler umfaßt.

7. Analytisches Element nach einem oder mehreren der Ansprüche 1 bis 6, dadurch gekennzeichnet, daß die Creatinase in einer solchen Menge vorhanden ist, daß ihre Aktivität das 1,2-fache oder mehr der Aktivität der Creatininase beträgt.

8. Analytisches Element nach einem oder mehreren der Ansprüche 1 bis 6, dadurch gekennzeichnet, daß die Creatinase in einer Menge von 6 000 IU/$m^2$ oder mehr und die Creatininase in einer Menge von 3 000 IU/$m^2$ oder mehr vorhanden sind.

9. Analytisches Element nach Anspruch 8, dadurch gekennzeichnet, daß die Creatinase in einer Menge von 6 000 IU/$m^2$ bis 2 500 000 IU/$m^2$ und die Creatininase in einer Menge von 5 000 IU/$m^2$ bis 500 000 IU/$m^2$ vorhanden sind.

10. Verfahren zum Analysieren von Creatinin in einer flüssigen Probe unter Verwendung eines mehrlagigen analytischen Elements nach Ansprüchen 1 und 2.